**Europäisches Patentamt**

(19) **European Patent Office**

**Office européen des brevets**

(11) Publication number : **0 333 831 B1**

# EUROPEAN PATENT SPECIFICATION

(45) Date of publication of patent specification :
15.07.92 Bulletin 92/29

(51) Int. Cl.⁵ : **A61K 31/555**

(21) Application number : **88908817.5**

(22) Date of filing : **06.09.88**

(86) International application number :
**PCT/US88/03052**

(87) International publication number :
**WO 89/02269 23.03.89 Gazette 89/07**

(54) USE OF METALLOPORPHYRINS TO REVERSE THE TOXIC EFFECT OF TUMOR THERAPY.

(30) Priority : **08.09.87 US 93817**

(43) Date of publication of application :
**27.09.89 Bulletin 89/39**

(45) Publication of the grant of the patent :
**15.07.92 Bulletin 92/29**

(84) Designated Contracting States :
**AT BE CH DE FR GB IT LI LU NL SE**

(56) References cited :
**US-A- 4 386 087**
**US-A- 4 668 670**
**US-A- 4 692 439**
**Chemical Abstracts, Volume 94 No 25, Issued 22 June 1981 (Columbus, Ohio, USA, Marchand et al., "Depression of CytochromeP-450-dependent drug Biotransformation by Adriamycin", see page 38, column 2, the Abstract No. 94: 202626k, Toxicol. Appl.Pharmacol. 1981, 58(1), 83-8 (Eng)).**
**Chemical Abstracts, Volume 93, No. 25, Issued 22 December 1980 (Columbus, Ohio USA, Ehninger et al. "Pharmacokinetics ofadriamycin and adriamycin metabolites", see page 20, column 1, the Abstract No. 93:230559p. Klin. Wochenschr. 1980, 58(18), 927-34(Ger)).**
**AM. J. DIS. Child, vol. 140, no. 2, February 1988, pages 147-150; A.M. POSSELT et al.: "Suppression of carbon monxideexcretion rate by tin protoporphyrin"**
**BIOCHEM. BIOPHYS. RES. COMMUN., vol. 150, no. 2, January 29, 1988, pages 822-827, Academic Press, Inc.; P.S. WISSEL et al.:"Tinprotoporphyrin inhibits heme oxygenase and prevents the decline in hepatic heme and cytochrome p-450 contents produced in nudemice by tumor transplantation"*The whole article***

(73) Proprietor : **THE ROCKEFELLER UNIVERSITY**
**1230 York Avenue**
**New York, NY 10021 (US)**

(72) Inventor : **KAPPAS, Attallah**
**1161 York Avenue - Apt. 4L**
**New York, NY 10021 (US)**
Inventor : **DRUMMOND, George, S.**
**304 West 7th Street**
**New York, NY 10023 (US)**
Inventor : **WISSEL, Paul, S.**
**100 Grove Avenue**
**Albany, NY 12208 (US)**

(74) Representative : **Weinhold, Peter, Dr. et al**
**Patentanwälte Dr. V. Schmied-Kowarzik**
**Dipl.-Ing. G. Dannenberg Dr. P. Weinhold Dr. D. Gudel Dipl.-Ing. S. Schubert Dr. P. BarzSiegfriedstrasse 8**
**W-8000 München 40 (DE)**

(56) References cited :

**PROCEEDINGS OF AACR, vol. 29, March 1988, page 20, abstract no. 79; P. WISSEL et al.:** "Tinprotoporphyrin (SnPPè) inhibitsheme oxygenase (HO) and prevents the decline in hepatic heme and cytochrome p-450 contents (p450) produced in nude mice by tumortransplantation" *Abstract*

**PROCEEDINGS OF AACR, vol. 29, March 1988, page 476, abstract no. 1891; P.S. WISSEL et al.:** "Protective effect ofSn-protoporhyrin against doxorubicin-induced pertubations of heme metabolism" *Abstract*

**PEDIATRIC RESEARCH, vol. 23, no. 1, January 1988, pages 50-53, International Pediatric Research Foundation, Inc., US; S.R.HINTZ et al.:** "Lack of inhibition of intestinal heme oxygenase by antibiotics and tinprotoporphyrin" *The whole article*

**THE JOURNAL OF BIOLOGICAL CHEMISTRY, vol. 257, no. 7, April 10, 1982, pages 3650-3654, US; C.S. CHANG et al.:** "Effects ofmetalloporphyrins on hemoglobin formation in mouse friend virus-transformed erythroleukemia cells"

**PROC. NATL: ACAD. SCI. USA, vol. 78, no. 10, October 1981, pages 6466-6470; G.S. DRUMMOND et al.:** "Prevention of neonatalhyperbilirubinemia by tin protoporphyrin IX, a potent competitive inhibitor of heme oxidation"

**CLIN. PHARMACOL. THER., vol. 39, May 1986, pages 510-520; K.B. ANDERSON et al.:** "Disposition of tin-protoporfphyrin andsuppression of hyperbilirubinemia in humans"

**Chemical Abstracts, vol. 101, no. 25, issued 17 December 1984 (Columbus, Ohio, USA, Kappas et al., "Control of heme andcytochrome P450 metabolims by inorganic metals, organometals, and synthetic metalloporphyrins", see page 220, column 2, theAbstract No. 101: 224234w, EPH, Environ. Health Perspect 1984. 57, 301-6 (Eng).**

## Description

The synthetic heme analog tin protoporphyrin-IX (SnPP) is a potent inhibitor of heme oxygenase, the rate limiting enzyme in the degradation of heme to bile pigment. In addition SnPP is also known as a complete suppressant of hyperbilirubinemia in neonatal mammals and for its ability to reduce plasma bilirubin levels in a variety of forms of naturally occurring or experimentally induced jaundice in animals and man.

Two analogs of SnPP are also known for the same activity. These are tin mesoporphyrin (SnMP) and tin diiododeuteroporphyrin ($SnI_2DP$).

It is known that the presence of tumors, whether spontaneous or implanted increases heme oxygenase activity, the rate limiting enzyme of heme catabolism, decreases -α aminolevulinate synthetase (ALA synthetase) activity, the rate limiting enzyme of heme synthesis, the concentration of microsomal heme, the concentration of cytochrome P-450 in liver and the activities of mixed function oxidases dependent on this heme protein. Various types of treatments for cancer, especially chemotherapy are known to increase these effects.

A large number of antineoplastic chemotherapeutic agents are known. Their activities are based on a variety of metabolic activities. These include alkylating agents such as N-alkyl-N-nitrosoureas and mitomycin-C, antimetabolites such as 6-mercaptopurine and 5-fluorouracil, inhibitors of protein synthesis such as anquidine and trichodermol, and anthracycline type antitumor agents which function by intercalation into DNA as well as alkylation, inhibition of protein synthesis, generation of free radicals and depletion of hepatic (and cardiac) glutathione stores. Intercalation is a process by which the antitumor agent moves into a separation between adjacent base pairs in the DNA helix causing profound changes in the properties of the intercalated DNA. Anthracycline type antineoplastic agents are, at this stage in the art, by far the most widely employed therapeutically useful products of this type. This class of antitumor antibiotic is clinically active in human leukemia, lymphoma (Hodgkins and Non-Hodgkins), breast, germ cell, lung (small cell), sarcoma, gastric and ovarian cancers.

The anthracyclines are a well known class of antitumor agents that have been employed in the clinical treatment of various tumors in humans since at least 1970. The best known of the class is doxorubicin. It has a wide spectrum of antitumor activity, such activity encompassing a broad range of solid tumors that prior to its isolation from <u>Streptomyces peucetius var caesius</u> had been relatively insensitive to chemotherapy, especially the soft tissue and bone sarcomas and bladder cancer. These activities of doxorubicin and other members of the class against various tumors have been discussed extensively by Carter in the Journal Of The National Cancer Institute, Vol. 55, No. 6, December 1975, pages 1265 to 1274.

Other anthracyclines, which have received clinical attention include daunorubicin, carminomycin and AD-32. Although there are other promising candidates, only doxorubicin and daunorubicin have, thus far, achieved widespread clinical acceptance.

The anthracyclines have been divided into two classes based on their selective effects on the inhibition of nucleic acid synthesis. Type I anthracyclines, exemplified by doxorubicin, daunorubicin and carminomycin inhibit DNA, whole cell RNA, and nucleolar RNA synthesis at approximately comparable concentrations, whereas Type II anthracyclines, exemplified by mytomycin-C aclacinomycin and marcellomycin inhibit whole cullular RNA synthesis at six- or seven-fold lower concentrations and nucleolar preribosomal synthesis at 170-1250-fold lower concentrations than those required for the inhibition of DNA synthesis. Both types have equivalent toxic effects. This invention is applicable to both types.

The anthracyclines are metabolized by cytochrome P-450 in the liver. Thus it is essential that normal levels of this heme protein be maintained in the liver.

It has been discovered that treatment of tumor bearing mammals with SnPP, SnMP or $SnI_2DP$ decreases heme oxygenase activity and normalizes ALA synthetase activity, hepatic heme and cytochome P-450 levels, thus reversing the toxic effects of cancer therapy or tumor burden. It has further been discovered that parenteral coadministration of these metalloporphyrins together with chemotherapeutic agents, especially anthracycline type antitumor agents such as doxorubicin will reverse the toxic effects of the chemotherapeutic agent on heme and cytochrome P-450. Coadministration of the metalloporphyrins and the chemotherapeutic agent means that the products are administered within a time frame in which the metallic porphyrins will exert their beneficial effects. This can mean several days, e.g. two to five days before initiation of chemotherapy. It can mean, and in preferred instances will mean, that the two agents are administered together, preferably in the same dosage unit.

Typically, chemotherapeutic treatment with agents such as doxorubicin follows an intense regimen in which the highest tolerable dosage is administered and continued until the toxic effects become so severe that it is necessary to decrease the dosage level or withhold treatment until the toxicity effects are substantially neutralized. At this point the cycle is repeated. The principal advantage of this invention is that it permits the use of the selected chemotherapeutic at higher levels for longer periods of time by maintaining normal levels of cytochrome P-450 and other related enzymes in the body tissues.

This invention, as will be apparent from the foregoing discussion comprises, in its broadest sense, a method of preventing the decline in heme and cytochrome P-450 in tumor bearing mammals, whether or not such mammals are undergoing tumor therapy, by administration to a tumor bearing patient in need of such treatment either because of an untreated tumor burden or because the mammal is undergoing antineoplastic therapy, an amount of SnPP, SnMP or $SnI_2DP$ which is effective to achieve the desired result. More specifically, it comprises a method of reversing the toxic effects caused by the presence of tumor burden whether or not there is chemotherapy, by administration to a patient a therapeutically effective amount of SnPP, SnMP or $SnI_2DP$ to cause the reversal of loss of hepatic heme and cytochrome associated with the tumor whether or not the patient is undergoing chemotherapy. The invention also includes therapeutic compositions for use in the practice of the methods.

The efficacy of the products of the invention for their newly discovered utility was established by experiments with three groups of male Sprague-Dawley rats (males, 150-200 grams, 16 per group). The rats were maintained on ad lib water and powdered rat chow (Purina) during the experiments. Group A was untreated, Group B was treated subcutaneously with doxorubicin alone on day 0, and Group C was treated with SnPP one day prior to doxorubicin which was administered on day 0.

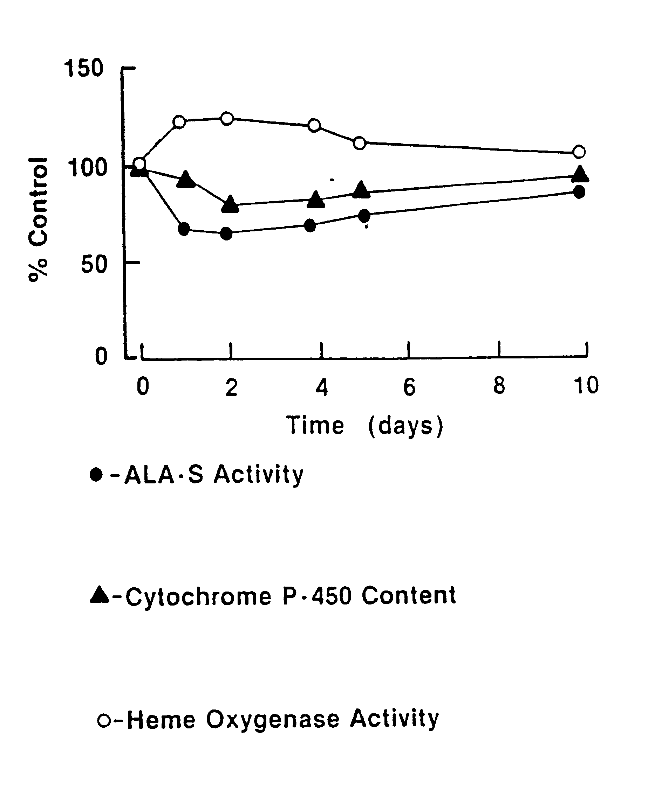

The effects of doxorubicin (10 mg/kg b.w.) on heme metabolism were studied with time and are shown in the figure. It will be seen that the increase in heme oxygenase activity and decrease in ALA synthetase activity and microsomal content of cytochrome P-450 were at a maximum on day 2 and returned to normal by day 10. Substantially the same results were observed at a doxorubicin dosage level of 5 mg/kg b.w.

As shown in Table 1, subcutaneous treatment of the rodents with 10 mg/kg b.w. doxorubicin produced a statistically significant ( $P < 0.05$) increase in hepatic heme oxygenase activity (20%) and a concomittant decrease in ALA synthetase activity (58%), and in the microsomal content of heme (45%) and cytochrome P-450 (18%) when these parameters were measured 2 days after doxorubicin administration. SnPP, when administered one day prior to doxorubicin, prevented the occurrence of these variations in heme metabolism. The group treated with doxorubicin and SnPP demonstrated a significant decrease (92%) in hepatic heme oxygenase activity and a significant increase (35%) in hepatic ALA-synthetase activity when compared to the group treated with doxorubicin alone. The levels of hepatic cytochrome P-450, and microsomal heme were substantially identical to those in the untreated group indicating that SnPP treatment prevented the detrimental effects of doxorubicin on heme metabolism. Similar effects are achieved with SnMP and $SnI_2PP$ and with other anthracycline type antitumor agents.

EP 0 333 831 B1

TABLE 1

HEME PROTEIN ANALYSIS ON DAY 2:

THE EFFECTS OF Sn-PP PRE-TREATMENT IN DOXORUBICIN TREATED RODENT

(DOX 10 mg/kg)

| | Cyt P-450 (nmol/mg protein) | Heme (nmol/mg protein) | ALA synthetase (nmol ALA formed/mg protein) | Heme oxygenase (nmol bilirubin formed/h /mg/protein |
|---|---|---|---|---|
| Control (A) | $0.71 \pm 0.05$ | $1.39 \pm 0.04$ | $0.27 \pm 0.01$ | $3.04 \pm 0.23$ |
| Doxorubicin alone (B) | $0.58 \pm 0.03*$ | $0.76 \pm 0.05*+$ | $0.17 \pm 0.01*$ | $3.65 \pm 0.32*+$ |
| Doxorubicin with Sn-PP (C) | $0.68 \pm 0.08$ | $1.56 \pm 0.02$ | $0.23 \pm 0.01$ | $0.30 \pm 0.11*+$ |

* statistically significant difference from control rodent, $p < 0.05$

+ statistically significant difference from Doxorubicin with Sn-PP pre-treatment

Mean $\pm$ SE   n = 16

The animals utilized in the tests were acclimated in metabolic cages maintained in a controlled environment with a 12h/12h light/dark cycle.

The compositions for parenteral administration were freshly prepared for each injection by addition of 0.4 N NaOH (20% v/v) to prepare a solution at pH 12, adjusted to pH 7.5 with 1 NHCl and made to final volume with 0.9% saline. The dosage level per injection was 50 $\mu$mol/kg b.w. Control animals were administered an equivalent amount of saline.

Animals were deprived of food but allowed free access to water for 16 hours prior to sacrifice. Livers were perfused in situ with ice-cold saline and homogenized in tris-HCL (0.01 M, pH 7.4) buffer containing sucrose (0.25 M). Homogenates (25% w/v) were centrifuged at 9000 g for 20 min, and the resulting mitochondrial pellet was used for the assay of ALA-synthetase activity. The 9000 xg supernatant was centrifuged at 105,000 xg for 60 min in a Beckman L5-50 ultracentrifuge. The microsomal pellet was resuspended in a minimum volume of potassium phosphate buffer (0.1 M, pH 7.4) and the cytosol was used as a source of biliverdin reductase. Cytochrome P-450 content was determined by the methods of Omura and Sato [J. Biol. Chem., 239: 2379 (1966) and 239: 2370 (1966)], and protein concentration by the method of Lowry et al [J. Biol. Chem., 193 265 (1951)] using crystalline bovine serum albumin as standard. Heme, ALA synthetase and heme oxygenase were assayed as described by Drummond and Kappas PNAS, 78, 6466-6470 (1981). Statistics were performed by Student's t-test (Prophet).

The products of the invention will be provided as parenteral compositions for injection. The selected metallic porphyrin or porphyrins can be suspended in an inert oil such as sesame, peanut or olive oil. Alternatively they can be suspended in an aqueous isotonic buffer at a pH of about 7 to 8, preferably 7.4 to 7.5. Useful buffers include sodium citrate-citric acid and sodium phosphate-phosphoric acid.

The desired isotonicity may be accomplished with sodium chloride or other pharmaceutically acceptable agents such as glucose, boric acid, sodium tartrate, propylene glycol or other organic or inorganic solutes. Sodium chloride and glucose are preferred because they are readily and inexpensively available in pure form.

The compositions, which may contain a metalloporphyrin as the principal active ingredient or a mixture of metalloporphyrin and chemotherapeutic agent such as doxorubicin, can also be prepared in lyophilized form for reconstitution in an aqueous medium such as isotonic saline or an isotonic buffer.

The attending physician will determine the optimum dose based on factors such as the selected metalloporphyrin, the age, weight, general health, tumor burden and other factors well known to those skilled in the art. The compositions will normally be provided in dosage unit form for one or a plurality of treatments. A dosage unit form for parenteral administration will typically contain from 7.15 to 71.5 mg/ml SnPP; 0.719 to 7.19 mg/ml SnMP; or 9.15 to 91.5 mg/ml SnI$_2$DP so as to provide dosages of from 0.715 to 7.15 mg/kg b.w.; 0.0719 to 7.19 mg/kg b.w.; and 0.915 to 9.15 mg/kg b.w. respectively. The amount of chemotherapeutic agent per dosage unit will, of course, vary with the selected agent, the clinical state of the patient and other factors which the physician in attendance can evaluate. For doxorubicin, each dosage unit as prepared for injection will contain about 20 to 70 mg/ml doxorubicin, typically as a pharmaceutically acceptable acid salt.

A dosage unit may also be provided as a container holding sufficient lyophilized powder to be reconstituted in an aqueous medium to contain the desired amounts of metalloporphyrin and, if desired, antineoplastic agent.

The therapeutic compositions of this invention will be prepared by the usual procedures employed for such purposes and need not be described here.

## Claims

1. A pharmaceutical composition for parenteral administration comprising a pharmaceutically acceptable carrier and, as the principal active ingredients SnPP, SnMP or SnI$_2$DP together with a chemotherapeutic agent.

2. A pharmaceutical composition as in claim 1 wherein the chemotherapeutic agent is an anthracycline type antitumor agent.

3. A pharmaceutical composition as in claim 2 wherein the anthracycline type antitumor agent is doxorubicin.

4. A pharmaceutical composition as in claims 1, 2 or 3 wherein the pharmaceutical carrier is buffered isotonic aqueous saline solution.

5. A pharmaceutical composition as in claims 1, 2 or 3 wherein the pharmaceutical carrier is buffered isotonic aqueous glucose solution.

6. A lyophilized composition comprising a chemotherapeutic agent and SnPP, SnMP or SnI$_2$PP.

7. A lyophilized composition as in claim 6 wherein the chemotherapeutic agent is an anthracycline type antitumor agent.

8. A lyophilized composition as in claim 7 wherein the anthracycline type antitumor agent is doxorubicin.

**Patentansprüche**

1. Pharmazeutische Zusammensetzung zur parenteralen Verabreichung, die einen pharmazeutisch annehmbaren Träger und als wesentliche aktive Bestandteile SnPP, SnMP oder $SnI_2DP$ zusammen mit einem chemotherapeutischen Mittel umfaßt.

2. Pharmazeutische Zusammensetzung nach Anspruch 1, worin das chemotherapeutische Mittel ein Antitumormittel vom Anthracyclintyp ist.

3. Pharmazeutische Zusammensetzung nach Anspruch 2, worin das Antitumormittel von Anthracyclintyp Doxorubicin ist.

4. Pharmazeutische Zusammensetzung nach den Ansprüchen 1, 2 oder 3, worin der pharmazeutische Träger gepufferte isotonische wäßrige Salzlösung ist.

5. Pharmazeutische Zusammensetzung nach den Ansprüchen 1, 2 oder 3, worin der pharmazeutische Träger gepufferte isotonische wäßrige Glucoselösung ist.

6. Lyophilisierte Zusammensetzung, die ein chemotherapeutisches Mittel und SnPP, SnMP oder $SnI_2PP$ umfaßt.

7. Lyophilsierte Zusammensetzung nach Anspruch 6, worin das chemotherapeutische Mittel ein Antitumormittel von Anthracyclintyp ist.

8. Lyophilisierte Zusammensetzung nach Anspruch 7, worin das Antitumormittel vom Anthracyclintyp Doxorubicin ist.


**Revendications**

1. Composition pharmaceutique pour l'administration parentérale, comprenant un support pharmaceutiquement acceptable et, en tant qu'ingrédients actifs principaux, SnPP, SnMP ou $SnI_2DP$, en même temps qu'un agent chimiothérapeutique.

2. Composition pharmaceutique selon la revendication 1, dans laquelle l'agent chimiothérapeutique est un agent antitumoral du type anthracycline.

3. Composition pharmaceutique selon la revendication 2, dans laquelle l'agent antitumoral du type anthracycline est la doxorubicine.

4. Composition pharmaceutique selon les revendications 1, 2 ou 3, dans laquelle le support pharmaceutique est une solution saline aqueuse isotonique tamponnée.

5. Composition pharmaceutique selon les revendications 1, 2 ou 3, dans laquelle le support pharmaceutique est une solution de glucose aqueuse isotonique tamponnée.

6. Composition lyophilisée comprenant un agent chimiothérapeutique et SnPP, SnMP ou $SnI_2DP$.

7. Composition lyophilisée selon la revendication 6, dans laquelle l'agent chimiothérapeutique est un agent antitumoral du type anthracycline.

8. Composition lyophilisée selon la revendication 7, dans laquelle l'agent antitumoral du type anthracycline est la doxorubicine.

●−ALA·S Activity

▲−Cytochrome P·450 Content

○−Heme Oxygenase Activity